# FASCICULE DE BREVET EUROPEEN

(11) **EP 0 850 939 B1**
(45) Date de publication et mention de la délivrance du brevet: **09.05.2001**
(21) Numéro de dépôt: 97403120.5
(22) Date de dépôt: 22.12.1997
(51) Int. Cl.: C07D 471/04, A61K 31/435

(54) **Dérivés d'ellipticine, leur procédé de préparation et les compositions pharmaceutiques qui les contiennent**
Ellipticinederivate, ihre Herstellung und diese enthaltende pharmazeutische Zubereitungen
Ellipticine derivatives, their preparation and pharmaceutical compositions containing them

(30) Priorité: 30.12.1996 FR 9616166
(43) Date de publication de la demande: 01.07.1998
(73) Titulaire: ADIR ET COMPAGNIE, 92415 Courbevoie Cédex (FR)
(72) Inventeur: Guillonneau, Claude, 92140 Clamart (FR); Bisagni, Emile, 91400 Orsay (FR); Charton, Yves, 92330 Sceaux (FR); Atassi, Ghanem, 92210 Saint Cloud (FR); Pierre, Alain, 78580 Les Alluets Le Roi (FR); Leonce, Stéphane, 78000 Versailles (FR)

(56) Documents cités:
- EP-A- 0 591 058

## Description

La présente invention concerne de nouveaux composés d'ellipticine, leur procédé de préparation et les compositions pharmaceutiques qui les contiennent. Les composés de la présente invention trouvent une utilisation thérapeutique intéressante grâce à leur activité antitumorale.

Des dérivés de l'ellipticine sont déjà connus pour leurs propriétés anticancéreuses. On peut citer à titre d'exemple le brevet EP 0 591 058 A1.

Les besoins de la thérapeutique exigent le développement constant de nouveaux anticancéreux dans le but d'obtenir des molécules à la fois plus actives et mieux tolérées.

La présente invention concerne des dérivés de l'ellipticine présentant notamment, par rapport aux composés de l'art antérieur, une plus grande cytotoxicité in vitro, ce qui laisse augurer d'une meilleure utilisation thérapeutique.

Plus particulièrement, la présente invention a pour objet des composés de formule générale I: 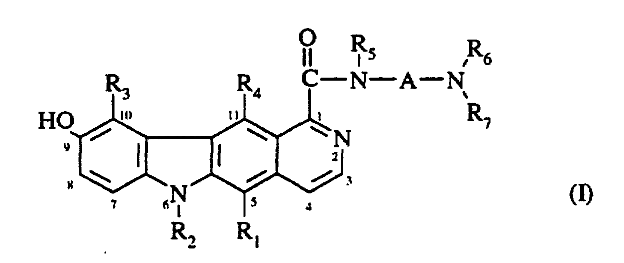 dans laquelle :
**R**_{**1**} représente un radical alkyle de 1 à 6 atomes de carbone en chaine droite ou ramifiée,
**R**_{**2**}, **R**_{**4**} **et R**_{**5**}, identiques ou différents, représentent chacun un atome d'hydrogène ou un radical (C₁-C₆)alkyle en chaine droite ou ramifiée ;
**R**_{**3**} représente :
   . un atome d'hydrogène,
   . un radical (C₁-C₆)alkyle en chaine droite ou ramifiée, éventuellement substitué, sur le carbone relié au tétracycle, par un groupe di(C₁-C₆)alkylarnino, ou
   . un radical (C₂-C₆)alkényle ;
**R**_{**6**} **et R**_{**7**}, identiques ou différents, représentent chacun un atome d'hydrogène ou un radical (C₁-C₆)alkyle, chacun en chaine droite ou ramifiée, et
R₆ et R₇ peuvent ensemble avec l'atome d'azote auquel ils sont liés former un radical hétérocyclique renfermant éventuellement un second hétéroatome choisi parmi les atomes d'azote, oxygène et soufre, tel que par exemple les radicaux pyrrolyle, pyrolinyle, pyrolidinyle, imidazolyle, imidazolinyle, imidazolidinyle, oxazolyle, oxazolinyle, oxazolidinyle, pyridyle, pipéridinyle, morpholinyle, thiomorpholinyle, pipérazinyle, pyrazolyle, pyrazolinyle. pyrazolidinyle, pyridazinyle, pyrimidinyle et pyrazinyle et de plus R₆ peut être lié à R₅ afin de former ensemble un pont -(CH₂)ₘ- dans lequel m prend les valeurs 2 ou 3 ; et

**A** représente une chaine hydrocarbonée saturée linéaire ou ramifiée contenant de 1 à 10 atomes de carbone,
à condition toutefois que A ne représente jamais une chaine hydrocarbonée saturée linéaire de 1 à 6 atomes de carbone lorsque simultanément R₃ prend la valeur hydrogène ;
ainsi que leurs éventuels isomères optiques, N-oxydes et sels d'addition, à un acide ou à une base, pharmaceutiquement acceptables.

La présente invention a également pour objet le procédé de préparation des composés de formule I, caractérisé en ce que
l'on fait réagir
. un composé de formule II : 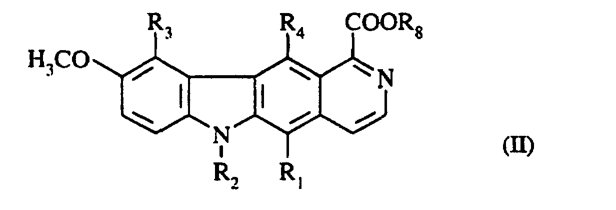 dans laquelle :
   - R₁, R₂, R₃ et R₄ ont les significations précédemment définies et,
   - R₈ représente un radical (C₁-C₆) alkyle en chaîne linéaire ou ramifiée,
. avec un hydracide, tel que par exemple l'acide chlorhydrique, ou avec un agent alcalin, tel que par exemple l'hydroxyde de sodium,
. pour obtenir un composé de formule III : 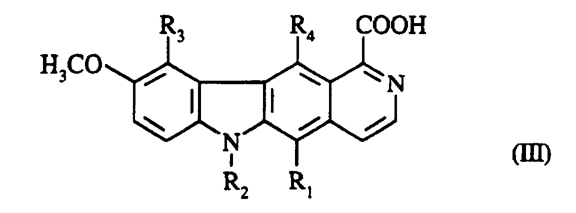 dans laquelle R₁, R₂, R₃ et R₄ ont les significations précédemment définies,
. que l'on fait réagir avec un composé de formule IV : 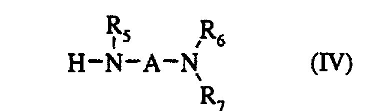 dans laquelle R₅, R₆, R₇ et A ont les significations précédemment définies,
. pour obtenir un composé de formule V : 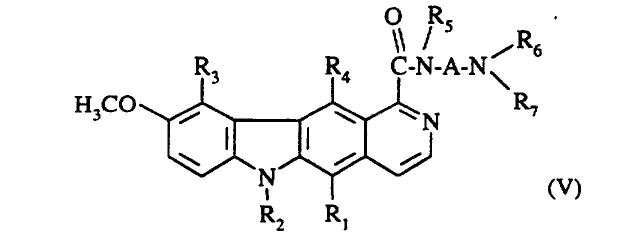 dans laquelle R₁, R₂, R₃, R₄, R₅, R₆, R₇, et A ont les significations précédemment définies,
. lequel est déméthylé au moyen d'un acide de Lewis, tel que par exemple le tribromure de bore,
pour donner le composé correspondant de formule I.

La saponification des composés II avec un agent alcalin tel que l'hydroxyde de sodium est réalisée avantageusement en opérant dans un solvant tel que l'éthanol aqueux.

La réaction du composé de formule III avec une amine de formule IV peut être effectuée avantageusement en présence d'un agent de couplage peptidique tel que le dicyclohexyl carbodiimide dans un solvant aprotique tel que le diméthylformamide, ou en présence de l'anhydride cyclique de l'acide 1-propanephosphonique (qui est un réactif commercial) dans un solvant aprotique tel que le diméthylformamide, selon la technique décrite par H.
WISSMANN et H.J. KLEINER, Angew. Chem. Int. Ed. Engl., 19, 133-134, (1980), ou en présence de l'hexafluorophosphate de benzotriazol-1-yloxytripyrrolidino phosphonium qui est un produit commercial, selon la technique décrite par J. COSTE, D. LE NGUYEN et B. CASTRO, Tetrahedron Letter, 31, 2, 205-208, (1990).

La déméthylation des composés de formule V peut s'effectuer de façon adéquate en présence d'un acide de Lewis tel que le tribromure de bore dans un solvant aprotique tel que le dichlorométhane ou le toluène pour conduire aux composés de formule I.

Selon les valeurs prises par les variables R₁ à R₇, les composés de formule I peuvent également être préparés selon d'autres variantes qui font toutes partie de la présente invention.

Ainsi, la présente invention a aussi pour objet le procédé de préparation des composés de formule I, caractérisé en ce que :
a) soit l'on fait réagir :
   . un composé de formule IIa : 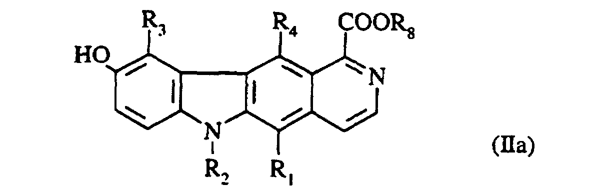 dans laquelle R₁, R₂, R₃, R₄, et R₈ ont les significations précédemment définies,
   . avec un composé de formule IVa : 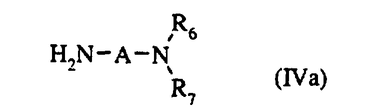 dans laquelle R₆, R₇ et A ont les significations précédemment définies,
   . pour obtenir un composé de formule Ia : 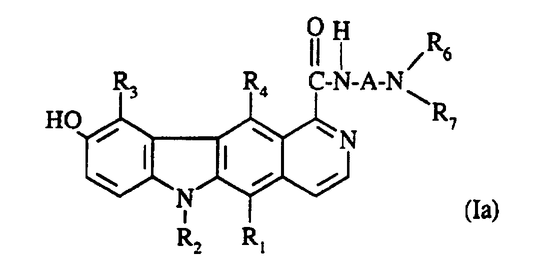 dans laquelle R₁, R₂, R₃, R₄, R₆, R₇ et A ont les significations précédemment définies,
b) soit l'on fait réagir :
   . un composé de formule Ib : 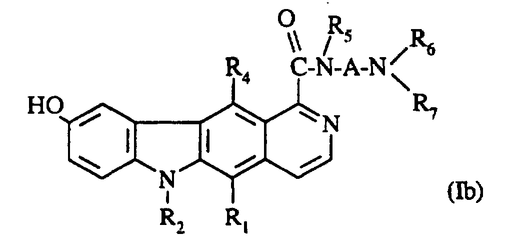 dans laquelle :
      - R₁, R₂, R₄, R₅, R₆, R₇ et A ont les significations précédemment définies,
   . soit avec un composé de formule VI : 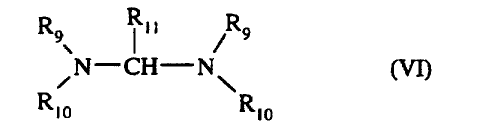 dans laquelle :
      - R₉ et R₁₀, identiques ou differents, représentent chacun un radical (C₁-C₆) alkyle linéaire ou ramifié, et
      - R₁₁ représente un radical de formule : CnH₂ₙ₊₁ dans laquelle n représente zéro ou un nombre entier de 1 à 5 inclus,
   . soit avec un composé de formule VII: 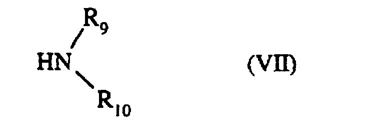 dans laquelle R₉ et R₁₀ ont les significations précédemment définies, en présence d'un aldéhyde contenant de 1 à 6 atomes de carbone,

   . pour obtenir un composé de formule Ic: 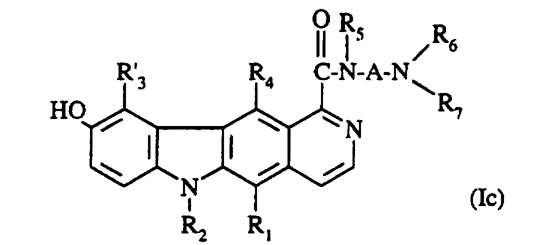 dans laquelle :
      - R₁, R₂, R₄, R₅, R₆, R₇ et A ont les significations précédemment définies et,
      - R'₃ représente : un radical (C₁-C₆) alkyle substitué, sur le carbone relié au tétracycle, par un groupe di (C₁-C₆) alkylamino (c'est-à-dire un radical : 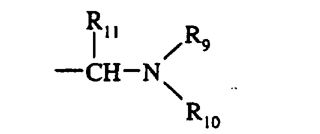 dans lequel R₉, R₁₀ et R₁₁ ont les significations précédemment définies) ;
c) soit l'on fait réagir :
   . un composé de formule Ic précédemment définie,
   . avec de l'hydrogène,
   . pour obtenir un composé de formule Id : 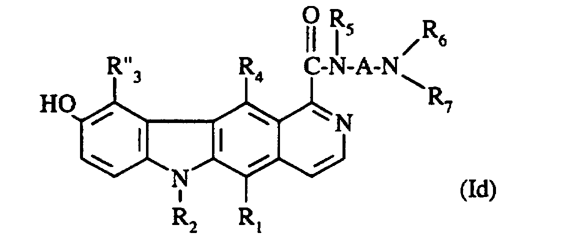 dans laquelle :
      - R₁, R₂, R₄, R₅, R₆, R₇ et A ont les significations précédemment définies et
      - R"₃ un radical (C₁-C₆) alkyle (soit le radical -CH₂-R₁₁ dans lequel R₁₁ à la signification précédemment définie) ;
d) soit l'on fait réagir
   . un composé de formule II'a : 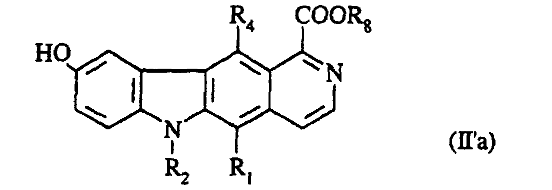 dans laquelle R₁, R₂, R₄ et R₈ ont les significations précédemment définies,
   . avec un composé de formule VIII :

      H₂C=CH-CH₂-X (VIII)

      dans laquelle :
      - X représente un groupe nucléofuge tel que par exemple un atome d'halogène ou un groupe alkylsulfonate ou arylsulfonate ;
   . pour obtenir un composé de formule IX : 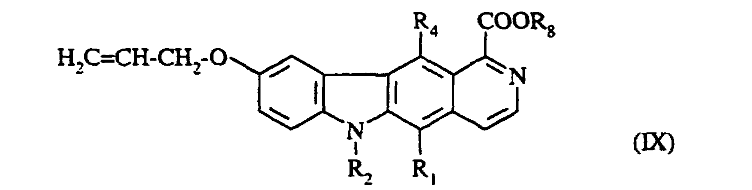 dans laquelle R₁, R₂, R₄ et R₈ ont les significations précédemment définies,
   . lequel par réarrangement thermique (transposition allylique) conduit au composé de formule IIb : 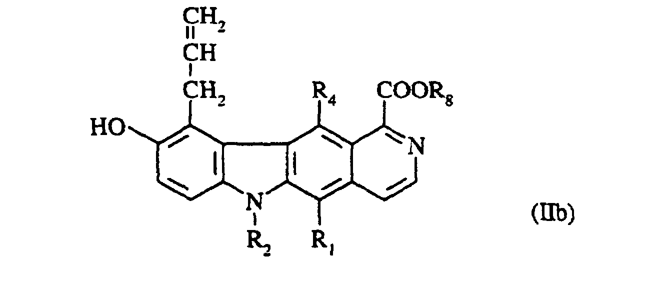 dans laquelle R₁, R₂, R₄ et R₈ ont les significations précédemment définies,
   . que l'on fait réagir avec une amine de formule IVa telle que précédemment définie,
   . pour obtenir un composé de formule Ie : 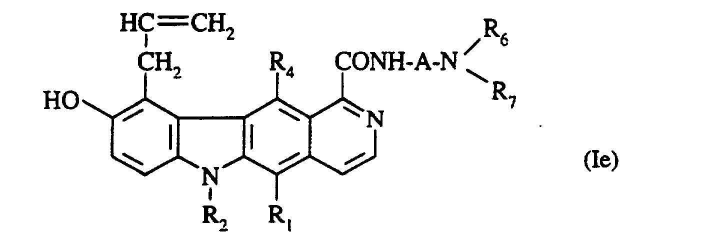 dans laquelle R₁, R₂, R₄, R₆, R₇ et A ont les significations précédemment définies ;
e) soit l'on hydrogène un composé de formule IIb précédemment définie pour obtenir un composé de formule IIc : 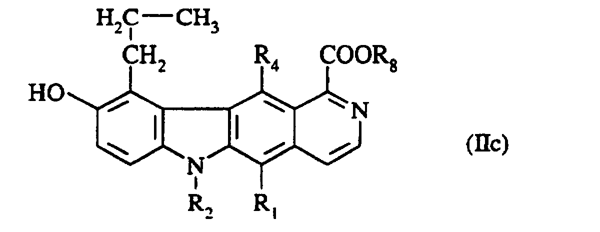 dans laquelle R₁, R₂, R₄ et R₈ ont les significations précédemment définies,
   . que l'on fait réagir avec une amine de formule IVa telle que précédemment définie,
   . pour obtenir un composé de formule If : 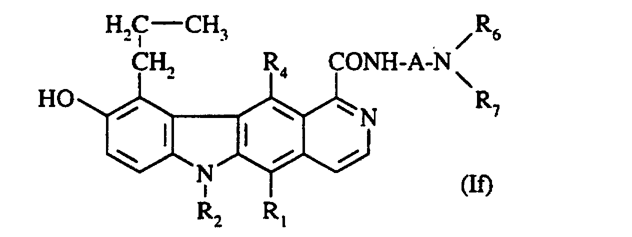 dans laquelle R₁, R₂, R₄, R₆, R₇ et A ont les significations précédemment définies.

Les composés de formule IIa, IIb et IIc appartiennent à l'ensemble des composés de formule II, de même que l'ensemble des composés de formule IVa fait partie de l'ensemble des composés de formule IV.

Les composés de formule Ia, Ib, Ic, Id, Ie et If sont tous inclus dans l'ensemble des composés de formule I.

Il est particulièrement avantageux de faire réagir un composé de formule IIa avec un composé de formule IVa, soit en utilisant un excès du composé de formule IVa, soit en opérant dans un solvant adéquate tel que par exemple un alcool à bas point d'ébullition ou un solvant aprotique comme le tétrahydrofurane ou le dichlorométhane, à une température comprise entre 80 à 150°C.

La réaction d'un composé de formule Ib avec un composé de formule VI peut être effectuée dans un solvant éthéré tel que le dioxane en présence d'un agent acide tel que l'acide acétique.

La réaction d'un composé de formule Ib avec un composé de formule VII s'effectue selon la technique décrite dans Organic Syntheses, Collective Volume IV, p 626.

L'hydrogénation catalytique des composés de formule Ic, pour obtenir les composés de formule Id, peut être effectuée en présence d'an catalyseur d'hydrogénation tel que le palladium sur charbon, le palladium sur sulfate de baryum ou l'hydroxyde de palladium sur charbon, éventuellement en présence d'un acide, par exemple en présence d'acide chlorhydrique dans un solvant à bas point d'ébullition, ou dans l'acide acétique.

La réaction des composés de formule II'a et VIII peut s'effectuer dans un solvant aprotique tel que le diméthylformamide, le tétrahydrofurane, l'acétone ou la pyridine, en présence ou non d'un accepteur d'hydracide tel qu'un carbonate alcalin comme le carbonate de potassium ou d'une amine tertiaire telle que la triéthylamine ou la diméthylaminopyridine.

La réaction de réarrangement allylique du composé de formule IX peut s'effectuer de façon adéquate dans un solvant aprotique à haut point d'ébullition tel que par exemple la diéthylamine ou le 1,2-dichlorobenzène, à la température d'ébullition du solvant utilisé.

L'hydrogénation catalytique du composé de formule IIb, pour obtenir un composé de formule IIc, peut être effectuée dans un alcool à bas point d'ébullition comme par exemple l'éthanol, à une température comprise entre 20 et 60°, sous une pression de 1.10⁵ à 5.10⁵ Pa, en présence d'un catalyseur d'hydrogénation tel que par exemple le palladium sur charbon ou le nickel de Raney.

Les méthodes de préparation des composés de formule II et IIa sont décrites dans le brevet EP-0591058 A1.

Les composés de formule IV sont soit des produits commerciaux soit des composés facilement accessibles par les méthodes classiques utilisées en chimie organique.

Les composés de formule I ainsi préparés peuvent être purifiés soit par chromatographie sous faible pression (chromato-flash) sur silice (AMICON 35-70 µ) en utilisant comme éluant par exemple l'acétate d'éthyle, un mélange de dichlorométhane et de méthanol, en présence ou non d'ammoniaque, soit par recristallisation des dits composés ou de leurs sels dans un solvant usuel tel que par exemple l'éthanol, l'eau ou le diméthylformamide.

Les composés de formule I donnent des sels avec des acides ou des bases physiologiquement tolérables, sels qui sont à ce titre inclus dans la présente invention.

Les composés de formule I donnent aussi des composés N-oxydes qui, à ce titre, sont également inclus dans la présente invention.

Certains composés de formule I renferment un ou plusieurs carbone asymétrique et de ce fait donnent des énantiomères ou des diastéréoisomères qui font également partie de la présente invention.

Les composés de la présente invention présentent des propriétés pharmacologiques particulièrement intéressantes, notamment une excellente cytoxicité in vitro et une bonne activité antitumorale, ce qui, ajouté au fait que ces composés sont particulièrement bien tolérés permet leur utilisation dans le traitement des cancers.

La présente invention a également pour objet les compositions pharmaceutiques contenant comme principe actif au moins un composé de la présente invention mélangé ou associé à un ou plusieurs excipients pharmaceutiques ou véhicules inertes non toxiques.

Ces compositions pharmaceutiques sont généralement présentée sous forme dosée convenant pour l'administration par voie orale, rectale ou parentérale et notamment les comprimés, dragées, gélules, suppositoires et solutions injectables ou buvables.

La posologie varie selon l'âge et le poids du patient, la voie d'administration, la nature de l'indication thérapeutique et les traitements éventuellement associés et s'échelonne entre 0,1 et 400 mg par jour en une ou plusieurs administrations.

Les exemples suivants illustrent la présente invention, les points de fusion étant déterminés à la platine chauffante de Kofler (K) ou au tube capillaire (cap).

### Exemple 1

### Le dichlorhydrate de 1-[(3-diméthylamino-2,2-diméthylpropyl)aminocarbonyl]-5,6-diméthyl-9-hydroxy-6H-pyrido[4,3-b]carbazole

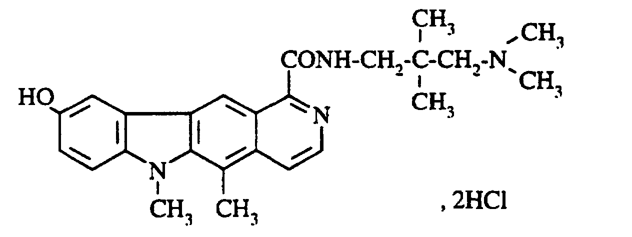

On agite à 130 °C pendant 20 heures 8 g de l-(méthoxycarbonyl)-5,6-diméthyl-9-hydroxy-6H-pyrido[4,3-b]carbazole dans 120 ml de N,N,2,2-tétraméthyl-1,3-propanediamine. On concentre à sec sous vide de pompe à palettes et on chromatographie le résidu (empaté sur 40 g de silice) sur 720 g de silice en éluant avec un mélange de dichlorométhane et d'éthanol (90-10). On concentre à sec les fractions pures et le résidu est mis en suspension dans 60 ml d'éthanol. On ajoute 20 ml d'éthanol chlorhydrique, observe une dissolution puis une cristallisation. On essore et sèche à 50 °C sous 66,6 Pa. On obtient 1 g de produit souhaité, PF(cap) : 275-280 °C. Rendement : 10 %.

### Exemple 2

### Trichlorhydrate de 1-[(2-diméthylaminoéthyl)aminocarbonyl]-5,6-diméthyl-9-hydroxy-10-diméthylaminométhyl-6H-pyrido[4,3-b]carbazole.

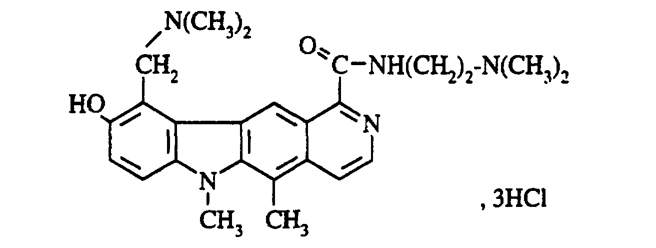

On porte au reflux pendant 30 minutes 0,9 g de 1-[(2-diméthylamino éthylaminocarbonyl)-5,6-diméthyl-9-hydroxy-6H-pyrido[4,3-b]carbazole, 3,25 g de NNN'N'-tétraméthyldiamino méthane, 0,3 ml d'acide acétique et 72 ml de dioxane. On concentre à sec. On reprend dans l'eau, neutralise par de l'ammoniaque concentrée et extrait au dichlorométhane en ajoutant de l'éthanol jusqu'à dissolution complète. La phase organique est séchée sur sulfate de magnésium puis évaporée à sec. On agite le résidu dans l'éther éthylique, essore et reprend dans l'éthanol. On ajoute de l'éthanol chlorhydrique jusqu'à pH < 3. On essore l'insoluble que l'on lave à l'éthanol, à l'éther, et que l'on sèche à 50 °C sous vide. On obtient 1,04 g de produit souhaité, PF(K) : > 250 °C. Rendement : 80 %.

### Exemple 3

### 1-[(2-diméthylaminoéthyl)aminocarbonyl]-5,6,10-triméthyl-9-hydroxy-6H-pyrido[4,3-b] carbazole

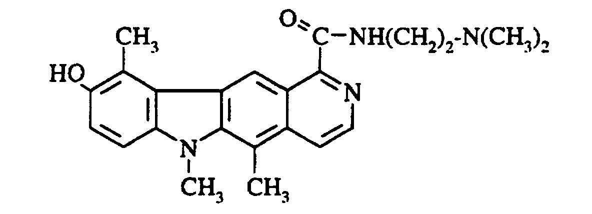

on hydrogène 2,6 g de 1-[(2-diméthylaminoéthyl)aminocarbonyl]-5,6-diméthyl-9-hydroxy-10-diméthylaminométhyl-6H-pyrido[4,3-b]carbazole dans 1,21 d'éthanol en présence de 2,2 g de Palladium sur charbon à 10 % à 30 °C pendant 4 heures (temps à partir duquel le produit de départ est devenu nettement minoritaire) sous une pression de 1.10⁵ Pa d'hydrogène. On filtre et lave le précipité avec un mélange de dichlorométhane et d'éthanol. Les filtrats joints sont concentrés à sec. On chromatographie le résidu sur silice en éluant d'abord avec un mélange de dichlorométhane et d'éthanol puis avec un mélange dichlorométhane, éthanol et triéthylamine. On obtient 0,23 g de produit souhaité, PF(K) : 170 °C. Rendement : 10 %.

### Exemple 4

### 1-[(2-diméthylaminoéthyl)aminocarbonyl]-5,6-diméthyl-9-hydroxy-10-allyl-6H-pyrido[4,3-b] carbazole

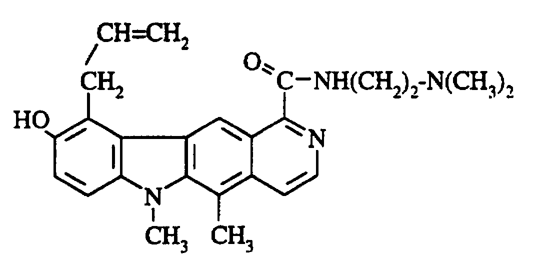

On dissous 2 g de 1-éthoxycarbonyl-5,6-diméthyl-9-allyloxy-6H-pyrido[4,3-b]carbazole dans 80 ml de 1,2-dichlorobenzène et on porte au reflux pendant 10 heures. On refroidit et on observe une précipitation. On essore et chromatographie le précipité sur silice en éluant d'abord avec du dichlorométhane puis avec un mélange de dichlorométhane et d'éthanol. On obtient 1,27 g de 1-éthoxycarbonyl-5,6-diméthyl-9-hydroxy-10-allyl-6H-pyrido[4,3-b]carbazole.

La transformation de ce composé en 1-[(2-diméthylaminoéthyl)aminocarbonyl]-5,6-diméthyl-9-hydroxy-10-allyl-6H-pyrido[4,3-b]carbazole a été réalisée en appliquant la méthode décrite dans l'exemple 1 avec un rendement de 75 %, PF(K) : 222 °C.

### Exemple 5

### 1-[(2-diméthylaminoéthyl)aminocarbonyl]-5,6-diméthyl-9-hydroxy-10-propyl-6H-pyrido[4,3-b]carbazole.

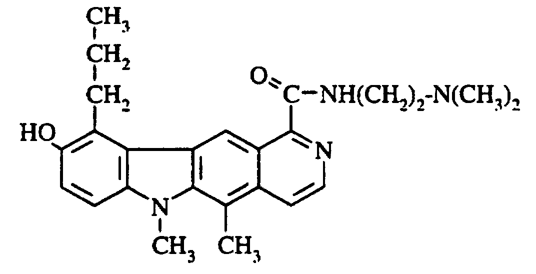

On hydrogène 0,7 g de 1-éthoxycarbonyl-5,6-diméthyl-9-hydroxy-10-allyl-6H-pyrido[4,3-b] carbazole dans 200 ml d'éthanol en présence de 0,3 g de Palladium sur charbon à 10 % à 40 °C sous une pression d'hydrogène de 1.10⁵ Pa pendant 30 minutes. On filtre, lave à l'éthanol le catalyseur et concentre à sec les filtrats joints. On chromatographie le résidu sur silice en éluant d'abord avec du dichlorométhane puis avec un mélange de dichlorométhane et d'éthanol (98-2). On obtient 0,362 g de 1-éthoxycarbonyl-5,6-diméthyl-9-hydroxy-10-propyl-6H-pyrido[4,3-b]carbazole. Rendement : 51 %.
La transformation de ce composé en 1-[(2-diméthylaminoéthyl)aminocarbonyl]-5,6-diméthyl-9-hydroxy-10-propyl-6H-pyrido[4,3-b]carbazole a été réalisée en appliquant la méthode décrite dans l'exemple 1 avec un rendement de 69 %, PF(K): 211 °C.

### Exemple 6 : ETUDE PHARMACOLOGIQUE

### Etude de cytotoxicité

Quatre lignées cellulaires ont été utilisées :
- 1 leucémie murine L 1210,
- 1 carcinome pulmonaire humain, A 549,
- 1 carcinome épidermoïde humain, KB-3-1,
- la lignée résistante correspondante, KB-A1, dont la résistance multidrogue a été induite par l'adriamycine (ADR).

Les cellules sont cultivées dans du milieu de culture RPMI 1640 complet contenant 10 % de sérum de veau foetal, 2 mM de glutamine, 50 unités/ml de pénicilline, 50 µg/ml de streptomycine et 10 mM d'Hepes, pH = 7,4.

Les cellules sont réparties dans des microplaques et exposées aux composés cytotoxiques. Les cellules sont ensuite incubées pendant 2 jours (L 1210) et 4 jours (A 549, KB-3-1, KB-A1). Le nombre de cellules viables est ensuite quantifié par un essai colorimétrique, le Microculture Tetrazolium Assay (Carmichael J., DeGraff W.G., Gazdar A.F., Minna J.D. and Mitchell J.R., Evaluation of a tetrazolium-based semiautomated colorimetric assay : assessment of chemosensitivity testing, Cancer Res., 47, 936-942, 1987).

Les résultats sont exprimés en IC50, concentration en cytotoxique qui inhibe à 50 % la prolifération des cellules traitées. Les résultats obtenus sur les lignées utilisées sont rassemblés dans le tableau ci-après.

A titre d'exemple sont répertoriés ci-après les résultats obtenus avec le composé de l'exemple 3 particulièrement représentatif de l'invention et l'Adriamycine (ADR) pris comme produit de référence.

| Composés testés | IC50,(nM) | | | |
|---|---|---|---|---|
| | L 1210 | A 549 | KB-3-1 | KB-A1 |
| Exemple 3 | 3,5 | 6,5 | 6,3 | 329,1 |
| ADR | 24,3 | 39,8 | 18,1 | 6746 |

La cytotoxicité du composé de l'exemple 3 est supérieure à celle de l'adriamycine sur les 4 lignées testées. Ce composé et tous les autres composés de la présente invention peuvent donc être utilisés avec succès contre des tumeurs résistantes à l'adriamycine et présentant le phénotype de résistance multidrogue.

## Revendications

1. Les composés de formule I : 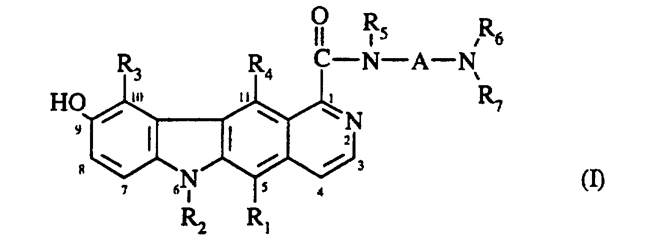 dans laquelle :
**R**_{**1**} représente un radical alkyle de 1 à 6 atomes de carbone en chaîne droite ou ramifiée,
**R**_{**2**}**, R**_{**4**} **et R**_{**5**}, identiques ou différents, représentent chacun un atome d'hydrogène ou un radical (C₁-C₆)alkyle en chaîne droite ou ramifiée ;
**R**_{**3**} représente :
. un atome d'hydrogène,
. un radical (C₁-C₆)alkyle en chaine droite ou ramifiée, éventuellement substitué, sur le carbone relié au tétracycle, par un groupe di(C₁-C₆)alkylamino, ou
. un radical (C₂-C₆)alkényle ;
**R**_{**6**} **et R**_{**7**}, identiques ou différents, représentent chacun un atome d'hydrogène ou un radical (C₁-C₆)alkyle, chacun en chaîne droite ou ramifiée, et R₆ et R₇ peuvent ensemble avec l'atome d'azote auquel ils sont liés former un radical hétérocyclique renfermant éventuellement un second hétéroatome choisi parmi les atomes d'azote, oxygène et soufre,
et de plus **R**_{**6**} peut être lié à **R**_{**5**} afin de former ensemble un pont -(CH₂)ₘ- dans lequel m prend les valeurs 2 ou 3 ; et
**A** représente une chaine hydrocarbonée saturée linéaire ou ramifiée contenant de 1 à 10 atomes de carbone,
à condition toutefois que A ne représente jamais une chaine hydrocarbonée saturée linéaire de 1 à 6 atomes de carbone lorsque simultanément R₃ prend la valeur hydrogène ; ainsi que leurs isomères optiques, N-oxydes et sels d'addition, à un acide ou à une base, pharmaceutiquement acceptables.

2. Un composé de la revendication 1 qui est le 1-[(2-diméthylarninoéthyl)aminocarbonyl]-5, 6, 10-triméthyl-9-hydroxy-6H-pyrido[4,3-b]carbazole.

3. Le procédé de préparation des composés de la revendication 1 caractérisé en ce que :
l'on fait réagir
. un composé de formule II : 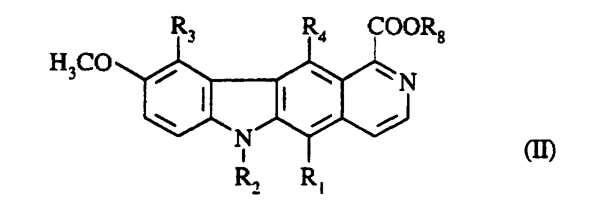 dans laquelle :
- R₁, R₂, R₃ et R₄ ont les significations définies dans la revendication 1 et,
- R₈ représente un radical (C₁-C₆) alkyle en chaine linéaire ou ramifiée,
. avec un hydracide, ou avec un agent alcalin,
. pour obtenir un composé de formule III : 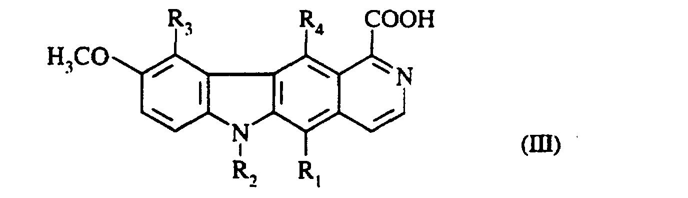 dans laquelle R₁, R₂, R₃ et R₄ ont les significations précédemment définies,
. que l'on fait réagir avec un composé de formule IV : 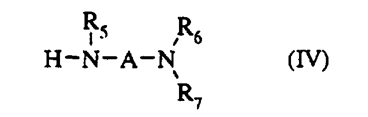 dans laquelle R₅, R₆, R₇ et A ont les significations définies dans la revendication 1,
. pour obtenir un composé de formule V : 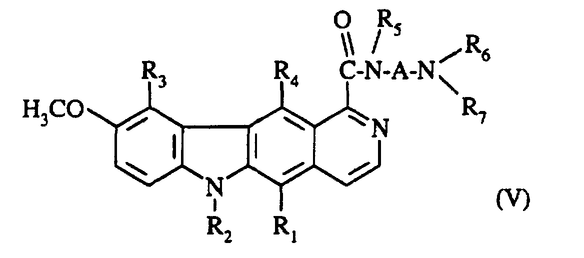 dans laquelle R₁, R₂, R₃, R₄, R₅, R₆, R₇, et A ont les significations précédemment définies,
. lequel est déméthylé au moyen d'un acide de Lewis,
pour donner le composé correspondant de formule I.

4. Le procédé de préparation des composés de la revendication 1, caractérisé en ce que :
a) soit l'on fait réagir :
. un composé de formule IIa : 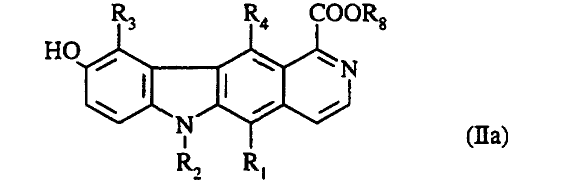 dans laquelle R₁, R₂, R₃, R₄, et R₈ ont les significations définies dans la revendication 3,
. avec un composé de formule IVa : 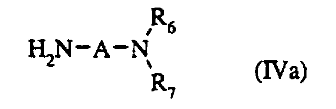 dans laquelle R₆, R₇ et A ont les significations définies dans la revendication 1,
. pour obtenir un composé de formule Ia : 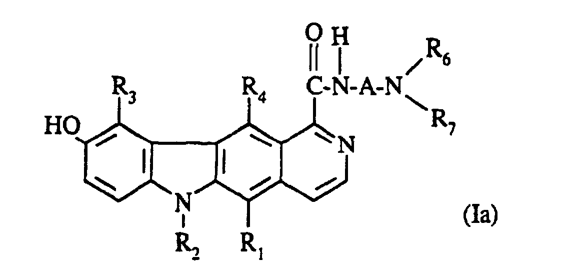 dans laquelle R₁, R₂, R₃, R₄, R₆, R₇ et A ont les significations précédemment définies.
b) soit l'on fait réagir :
. un composé de formule Ib : 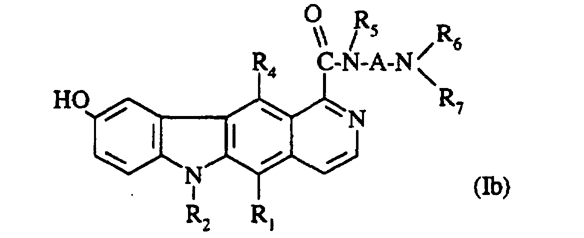 dans laquelle :
- R₁, R₂, R₄, R₅, R₆, R₇ et A ont les significations définies dans la revendication 1,
. soit avec un composé de formule VI : 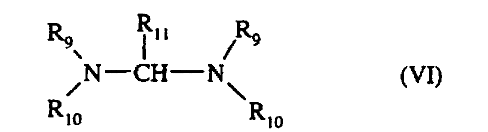 dans laquelle :
- R₉ et R₁₀, identiques ou differents, représentent chacun un radical (C₁-C₆) alkyle linéaire ou ramifié, et
- R₁₁ représente un radical de formule : CnH₂ₙ₊₁ dans laquelle n représente zéro ou un nombre entier de 1 à 5 inclus,
. soit avec un composé de formule VII : 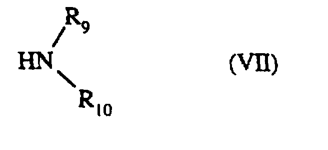 dans laquelle R₉ et R₁₀ ont les significations précédemment définies, en présence d'un aldéhyde contenant de 1 à 6 atomes de carbone,
. pour obtenir un composé de formule Ic : 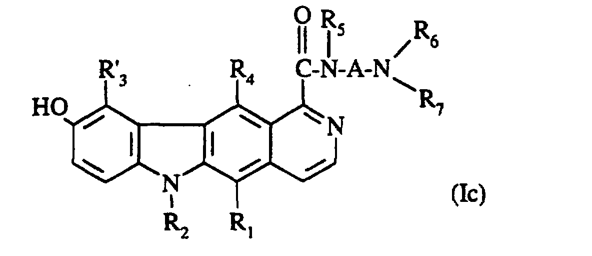 dans laquelle :
- R₁, R₂, R₄, R₅, R₆, R₇ et A ont les significations précédemment définies et,
- R'₃ représente : un radical (C₁-C₆) alkyle substitué, sur le carbone relié au tétracycle, par un groupe di (C₁-C₆) alkylamino (c'est-à-dire un radical : 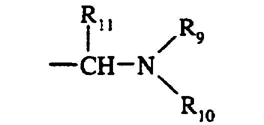 dans lequel R₉, R₁₀ et R₁₁ ont les significations précédemment définies) ;
c) soit l'on fait réagir :
. un composé de formule Ic précédemment définie,
. avec de l'hydrogène,
. pour obtenir un composé de formule Id : 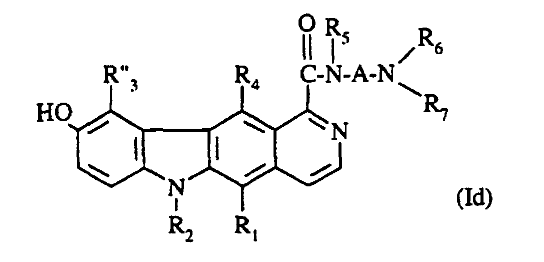 dans laquelle :
- R₁, R₂, R₄, R₅, R₆, R₇ et A ont les significations précédemment définies et
- R"₃ un radical (C₁-C₆) alkyle (soit le radical -CH₂-R₁₁ dans lequel R₁₁ à la signification précédemment définie) ;
d) soit l'on fait réagir
. un composé de formule II'a : 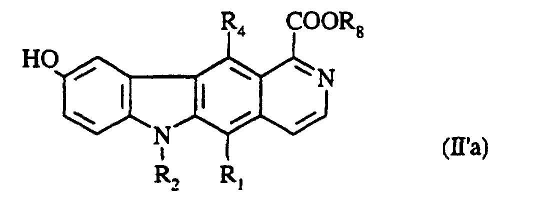 dans laquelle R₁, R₂, R₄ et R₈ ont les significations précédemment définies,
. avec un composé de formule VIII :
H₂C=CH-CH₂-X (VIII)
dans laquelle :
- X représente un groupe nucléofuge,
. pour obtenir un composé de formule IX : 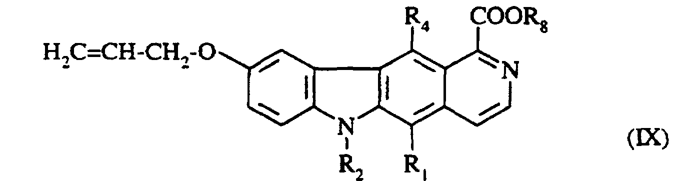 dans laquelle R₁, R₂, R₄ et R₈ ont les significations précédemment définies,
. lequel par réarrangement thermique (transposition allylique) conduit au composé de formule IIb : 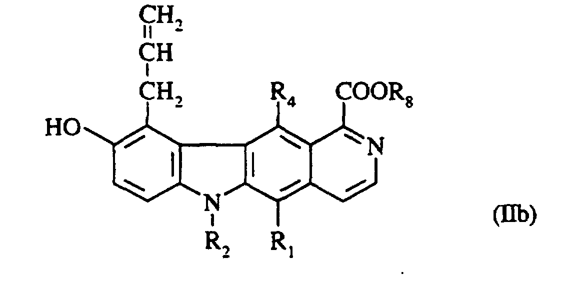 dans laquelle R₁, R₂, R₄ et R₈ ont les significations précédemment définies,
. que l'on fait réagir avec une amine de formule IVa telle que précédemment définie,
. pour obtenir un composé de formule Ie : 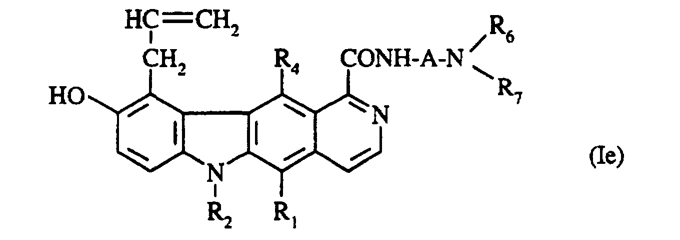 dans laquelle R₁, R₂, R₄, R₆, R₇ et A ont les significations précédemment définies ;
e) soit l'on hydrogène un composé de formule IIb précédemment définie pour obtenir un composé de formule IIc : 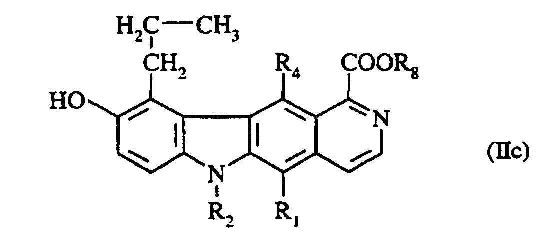 dans laquelle R₁, R₂, R₄ et R₈ ont les significations précédemment définies,
. que l'on fait réagir avec une amine de formule IVa telle que précédemment définie,
. pour obtenir un composé de formule If : 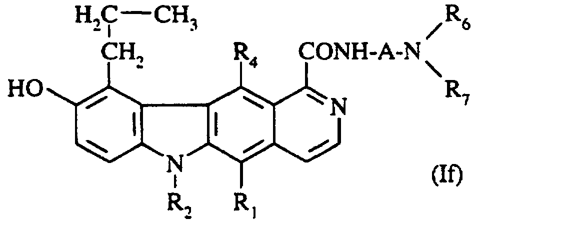 dans laquelle R₁, R₂, R₄, R₆, R₇ et A ont les significations précédemment définies.

5. Les compositions pharmaceutiques contenant comme principe actif au moins un composé selon l'une quelconque des revendications 1 et 2, mélangé ou associé à un ou plusieurs excipients pharmaceutiques ou véhicules inertes non toxiques.

6. Les compositions pharmaceutiques selon la revendication 5, utiles dans le traitement des cancers.

## Patentansprüche

1. Verbindungen der Formel I: 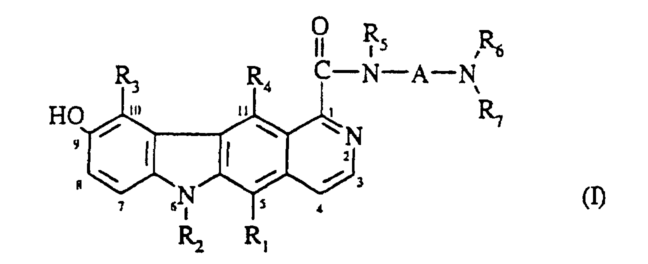 in der:
**R**_{**1**} eine geradkettige oder verzweigte Alkylgruppe mit 1 bis 6 Kohlenstoffatomen bedeutet,
**R**_{**2**}**, R**_{**4**} **und R**_{**5**}**,** die gleichartig oder verschieden sind, jeweils ein Wasserstoffatom oder eine geradkettige oder verzweigte (C₁-C₆)-Alkylgruppe bedeuten; **R**_{**3**}**:**
. ein Wasserstoffatom,
. eine geradkettige oder verzweigte (C₁-C₆)-Alkylgruppe, die gegebenenfalls am mit dem Tetrazyklus verbundenen Kohlenstoffatom durch eine Di-(C₁-C₆)-al-kylaminogruppe substituiert ist, oder
. eine (C₂-C₆)-Alkenylgruppe bedeutet;
**R**_{**6**} **und R**_{**7**}**,** die gleichartig oder verschieden sind, jeweils ein Wasserstoffatom oder eine geradkettige oder verzweigte (C₁-C₆)-Alkylgruppe darstellen und
R₆ und R₇ gemeinsam mit dem Stickstoffatom, an das sie gebunden sind, eine heterocyclische Gruppe bilden können, die gegebenenfalls ein zweites Heteroatom ausgewählt aus Stickstoff-, Sauerstoff- und Schwefelatomen enthalten kann,
und weiterhin **R**_{**6**} mit **R**_{**5**} verbunden sein kann unter gemeinsamer Ausbildung einer Brücke der Formel -(CH₂)ₘ-, in der m die Werte 2 oder 3 besitzt; und
**A** eine gesättigte geradkettige oder verzweigte Kohlenwasserstoffkette, die 1 bis 10 Kohlenstoffatome enthält, bedeutet,
mit der Maßgabe, daß A keine gesättigte geradkettige Kohlenwasserstoffkette mit 1 bis 6 Kohlenstoffatomen darstellt, wenn gleichzeitig R₃ ein Wasserstoffatom bedeutet,
sowie deren optische Isomere, N-Oxide und Additionssalze mit einer pharmazeutisch annehmbaren Säure oder Base.

2. Verbindung nach Anspruch 1, nämlich 1-[(2-Dimethylaminoethyl)-amino-carbonyl]-5,6,10-trimethyl-9-hydroxy-6H-pyrido(4,3-b)carbazol.

3. Verfahren zur Herstellung der Verbindungen nach Anspruch 1, dadurch gekennzeichnet, daß man:
. eine Verbindung der Formel II: 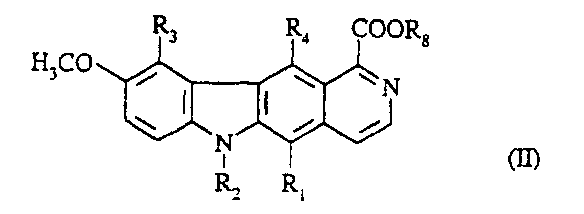 in der:
- R₁, R₂, R₃ und R₄ die in Anspruch 1 angegebenen Bedeutungen besitzen und
- R₈ eine geradkettige oder verzweigte (C₁-C₆)-Alkylgruppe darstellt,
. mit einer Wasserstoffsäure oder mit einem alkalischen Mittel umsetzt,
. zur Bildung einer Verbindung der Formel III: 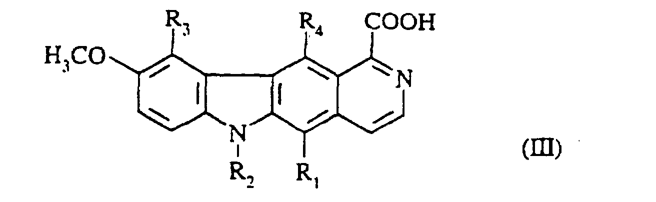 in der R₁, R₂, R₃ und R₄ die oben angegebenen Bedeutungen besitzen,
. welche man mit einer Verbindung der Formel IV: 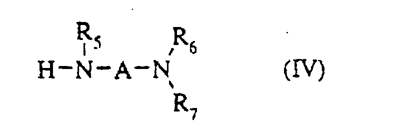 in der R₅, R₆, R₇ und A die in Anspruch 1 angegebenen Bedeutungen besitzen, umsetzt
. zur Bildung einer Verbindung der Formel V: 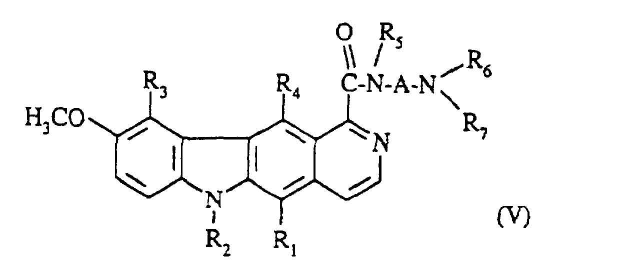 in der R₁, R₂, R₃, R₄, R₅, R₆, R₇ und A die oben angegebenen Bedeutungen besitzen,
. welche mit Hilfe einer Lewis-Säure demethyliert wird, zur Bildung der entsprechenden Verbindung der Formel I.

4. Verfahren zur Herstellung der Verbindungen nach Anspruch 1, dadurch gekennzeichnet, daß man:
a) entweder:
. eine Verbindung der Formel IIa: 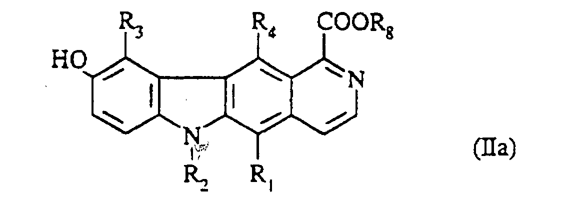 in der R₁, R₂, R₃, R₄ und R₈ die in Anspruch 3 angegebenen Bedeutungen besitzen,
. mit einer Verbindung der Formel IVa: 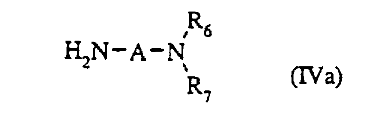 in der R₆, R₇ und A die in Anspruch 1 angegebenen Bedeutungen besitzen, umsetzt,
. zur Bildung der Verbindung der Formel Ia: 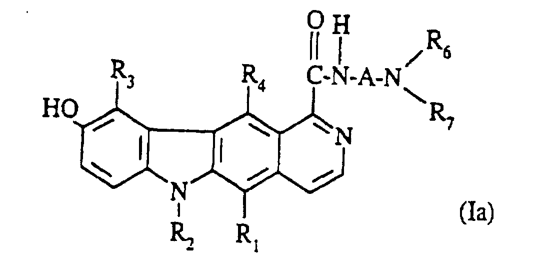 in der R₁, R₂, R₃, R₄, R₆, R₇ und A die oben angegebenen Bedeutungen besitzen,
b) oder:
. eine Verbindung der Formel Ib: 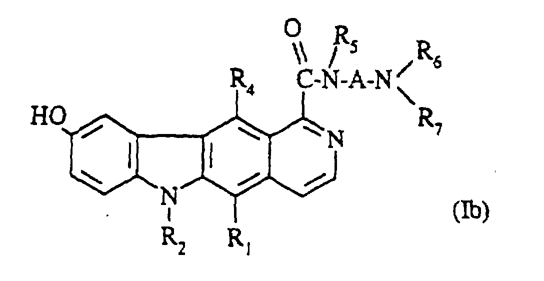 in der:
- R₁, R₂, R₄, R₅, R₆, R₇ und A die in Anspruch 1 angegebenen Bedeutungen besitzen,
. entweder mit einer Verbindung der Formel VI: 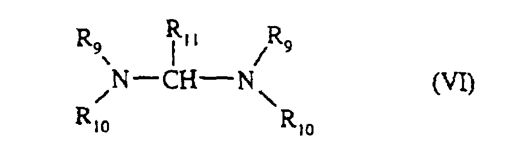 in der:
- R₉ und R₁₀, die gleichartig oder verschieden sind, jeweils eine geradkettige oder verzweigte (C₁-C₆)-Alkylgruppe darstellen und
- R₁₁ eine Gruppe der Formel CₙH₂ₙ₊₁. in der n Null oder eine Zahl zwischen 1 und 5 einschließlich bedeutet, darstellt,
. oder mit einer Verbindung der Formel VII: 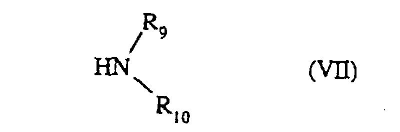 in der R₉ und R₁₀ die oben angegebenen Bedeutungen besitzen,
in Gegenwart eines Aldehyds, der 1 bis 6 Kohlenstoffatome enthält, umsetzt,
. zur Bildung einer Verbindung der Formel Ic: 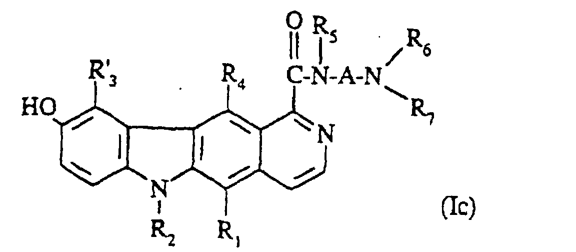 in der:
- R₁, R₂, R₄, R₅, R₆, R₇ und A die oben angegebenen Bedeutungen besitzen und
- R'₃ eine (C₁-C₆)-Alkylgruppe darstellt, die am mit dem Tetrazyklus verbundenen Kohlenstoffatom durch eine Di-(C₁-C₆)-alkylaminogruppe substituiert ist (d. h. eine Gruppe: 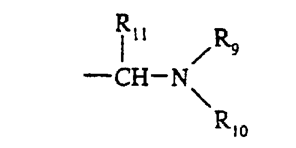 in der R₉, R₁₀ und R₁₁ die oben angegebenen Bedeutungen besitzen) darstellt;
c) oder:
. eine Verbindung der oben definierten Formel Ic
. mit Wasserstoff umsetzt
. zur Bildung einer Verbindung der Formel Id: 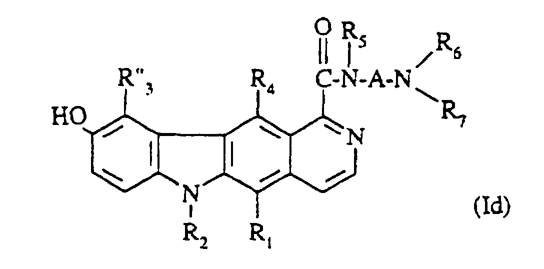 in der:
- R₁, R₂, R₄, R₅, R₆, R₇ und A die oben angegebenen Bedeutungen besitzen und
- R''₃ eine (C₁-C₆)-Alkylgruppe (d. h. die Gruppe -CH₂-R₁₁, worin R₁₁ die oben angegebenen Bedeutungen besitzt) darstellt;
d) oder man
. eine Verbindung der Formel II'a: 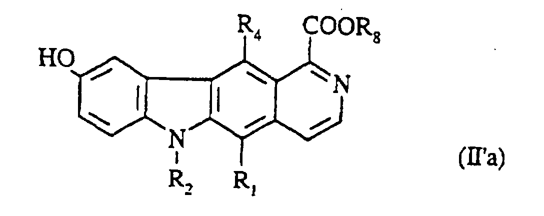 in der R₁, R₂, R₄ und R₈ die oben angegebenen Bedeutungen besitzen, mit einer Verbindung der Formel VIII:
H₂C=CH-CH₂-X (VIII)
in der:
- X eine nucleofuge Gruppe darstellt, umsetzt,
. zur Bildung einer Verbindung der Formel IX: 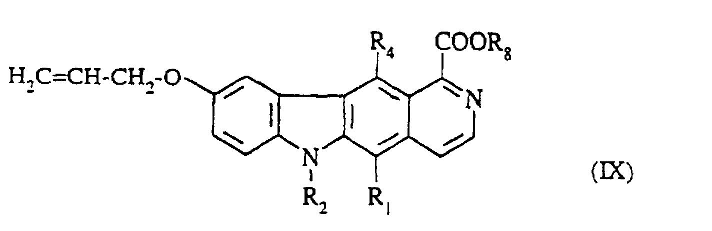 in der R₁, R₂, R₄ und R₈ die oben angegebenen Bedeutungen besitzen,
. welche durch thermische Umlagerung (Allylumlagerung) zu der Verbindung der Formel IIb führt: 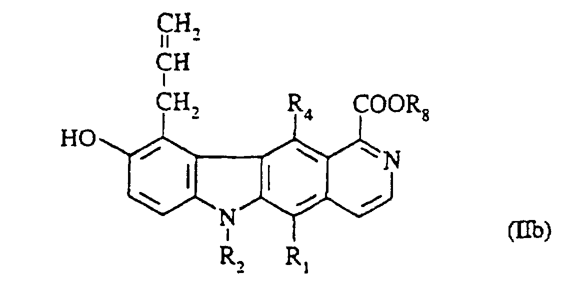 in der R₁, R₂, R₄ und R₈ die oben angegebenen Bedeutungen besitzen,
. welche man mit einem Amin der Formel IVa, wie sie oben definiert worden ist, umsetzt
. zur Bildung einer Verbindung der Formel Ie: 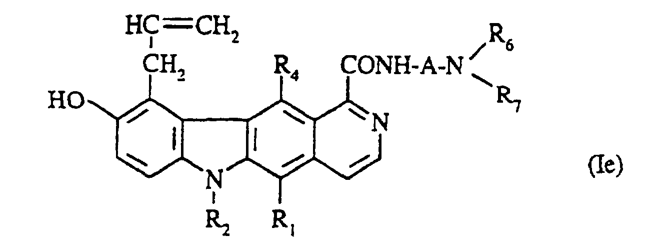 in der R₁, R₂, R₄, R₆, R₇ und A die oben angegebenen Bedeutungen besitzen;
e) oder man eine Verbindung der oben definierten Formel IIb hydriert zur Bildung einer Verbindung der Formel IIc: 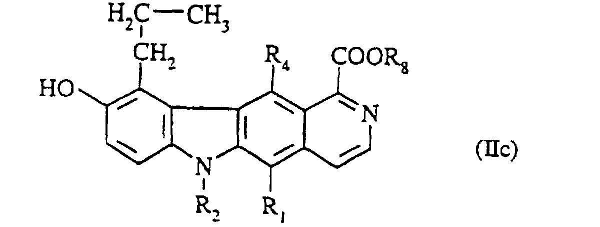 in der R₁, R₂, R₄ und R₈ die oben angegebenen Bedeutungen besitzen,
. welche man mit einem Amin der oben definierten Formel IVa umsetzt
. zur Bildung einer Verbindung der Formel If: 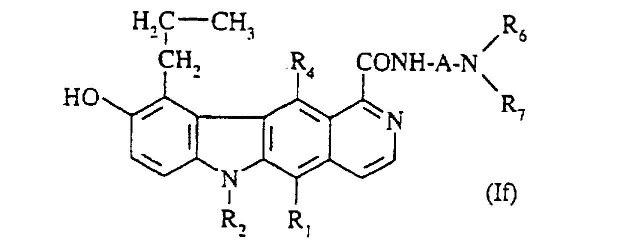 in der R₁, R₂, R₄, R₆, R₇ und A die oben angegebenen Bedeutungen besitzen.

5. Pharmazeutische Zubereitungen enthaltend als Wirkstoff mindestens eine Verbindung nach einem der Ansprüche 1 und 2 in Mischung oder in Kombination mit einem oder mehreren inerten, nichttoxischen, pharmazeutischen Trägermaterialien oder Bindemitteln.

6. Pharmazeutische Zubereitungen nach Anspruch 5 zur Behandlung von Krebserkrankungen.

## Claims

1. Compounds of formula I: 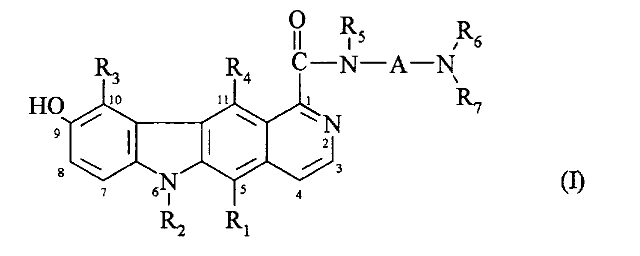 in which:
**R**_{**1**} represents a straight- or branched-chained alkyl radical having from 1 to 6 carbon atoms;
**R**_{**2**}, **R**_{**4**} **and R**_{**5**}**,** which may be the same or different, each represents a hydrogen atom or a straight- or branched-chained (C₁-C₆)alkyl radical;
**R**_{**3**} represents:
. a hydrogen atom,
. a straight- or branched-chained (C₁-C₆)alkyl radical which is optionally substituted, on the carbon atom bonded to the tetracycle, by a di(C₁-C₆)alkylamino group, or
. a (C₂-C₆)alkenyl radical;
**R**_{**6**} **and R**_{**7**}, which may be the same or different, each represents a hydrogen atom or a (C₁-C₆)alkyl radical, in each case in a straight or branched chain, and
R₆ and R₇ may, together with the nitrogen atom to which they are bonded, form a heterocyclic radical which optionally contains a second hetero atom selected from the atoms nitrogen, oxygen and sulphur,
and, furthermore, **R**_{**6**} may be bonded to **R**_{**5**} in order to form together a bridge -(CH₂)ₘ-in which m has the value 2 or 3; and
**A** represents a saturated linear or branched hydrocarbon chain containing from 1 to 10 carbon atoms,
with the proviso, however, that A does not represent a saturated linear hydrocarbon chain having from 1 to 6 carbon atoms when, simultaneously, R₃ is hydrogen;
as well as their optical isomers, N-oxides and pharmaceutically acceptable addition salts with an acid or a base.

2. A compound of claim 1 which is 1-[(2-dimethylaminoethyl)aminocarbonyl]-5,6,10-trimethyl-9-hydroxy-6H-pyrido[4,3-b]carbazole.

3. Process for the preparation of compounds of claim 1, characterised in that:
. a compound of formula II: 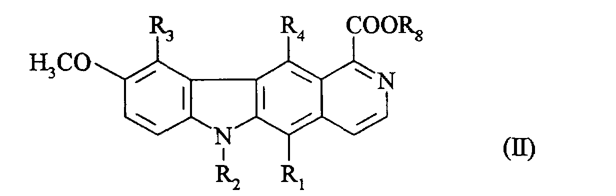 in which:
- R₁, R₂, R₃ and R₄ are as defined in claim 1, and
- R₈ represents a straight- or branched-chained (C₁-C₆)alkyl radical,
is reacted
. with a hydracid or with an alkaline agent
. to give a compound of formula III: 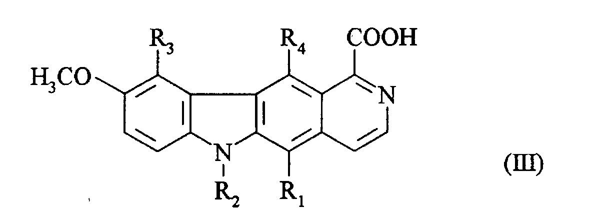 in which R₁, R₂, R₃ and R₄ are as defined above,
. which is reacted with a compound of formula IV: 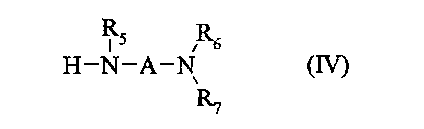 in which R₅, R₆, R₇ and A are as defined in claim 1,
. to give a compound of formula V: 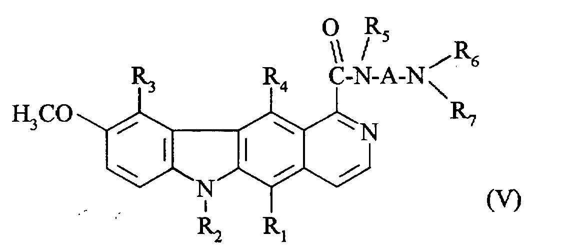 in which R₁, R₂, R₃, R₄, R₅, R₆, R₇ and A are as defined above,
. which is demethylated by means of a Lewis acid
to yield the corresponding compound of formula I.

4. Process for the preparation of compounds of claim I, characterised in that:
a) a compound of formula IIa: 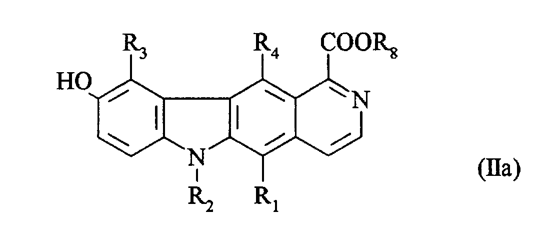 in which R₁, R₂, R₃, R₄ and R₈ are as defined in claim 3, is reacted
. with a compound of formula IVa: 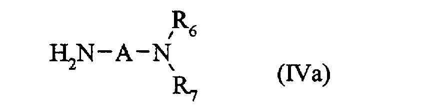 in which R₆, R₇ and A are as defined in claim 1,
. to give a compound of formula Ia: 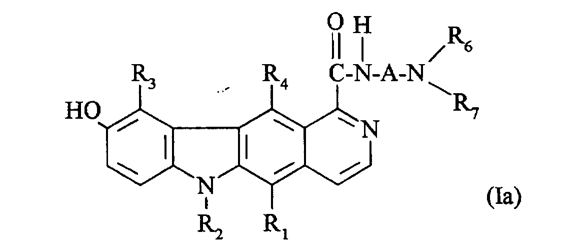 in which R₁, R₂, R₃, R₄, R₆, R₇ and A are as defined above; or
b) a compound of formula Ib: 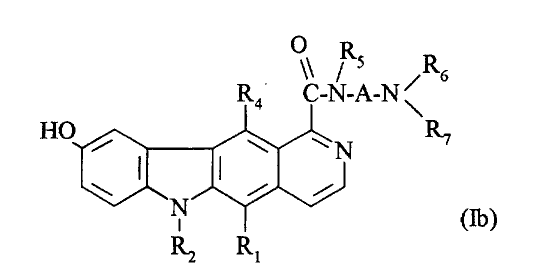 in which R₁, R₂, R₄, R₅, R₆, R₇ and A are as defined in claim 1, is reacted
. either with a compound of formula VI: 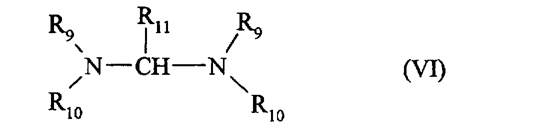 in which:
- R₉ and R₁₀, which may be the same or different, each represents a linear or branched (C₁-C₆)alkyl radical, and
- R₁₁ represents a radical of the formula : CnH₂ₙ₊₁ in which n represents zero or an integer from 1 to 5 inclusive,
. or with a compound of formula VII: 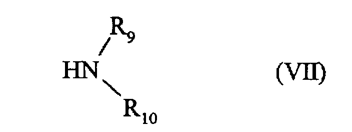 in which R₉ and R₁₀ are as defined above,
in the presence of an aldehyde containing from 1 to 6 carbon atoms,
. to give a compound of formula Ic: 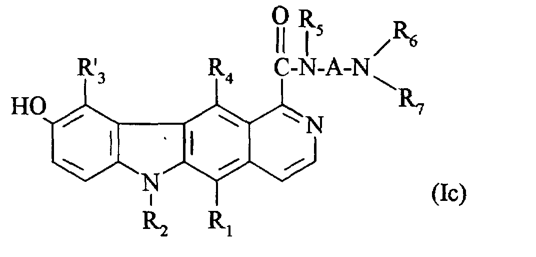 in which:
- R₁, R₂, R₄, R₅, R₆, R₇ and A are as defined above, and
- R'₃ represents : a (C₁-C₆)alkyl radical which is substituted, on the carbon atom bonded to the tetracycle, by a di(C₁-C₆)alkylamino group (i.e. a radical: 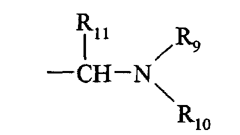 in which R₉, R₁₀ and R₁₁ are as defined above); or
c) a compound of formula Ic defined above is reacted
. with hydrogen
. to give a compound of formula Id: 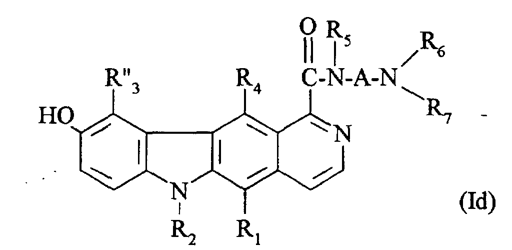 in which:
- R₁, R₂, R₄, R₅, R₆, R₇ and A are as defined above, and
- R"₃ represents a (C₁-C₆)alkyl radical (i.e. the radical -CH₂-R₁₁ in which R₁₁ is as defined above);
or
d) a compound of formula II'a: 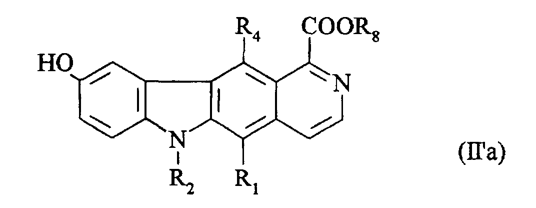 in which R₁, R₂, R₄ and R₈ are as defined above,
is reacted
. with a compound of formula VIII:
H₂C=CH-CH₂-X (VIII)
in which X represents a nucleofugal group,
. to give a compound of formula IX: 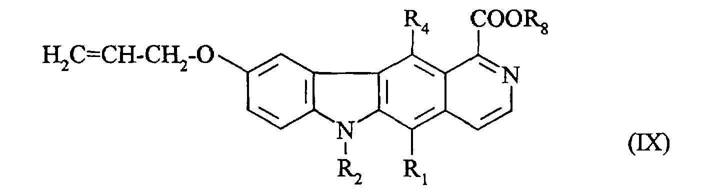 in which R₁, R₂, R₄ and R₈ are as defined above,
. which, by means of thermal rearrangement (allyl transposition), yields the compound of formula IIb: 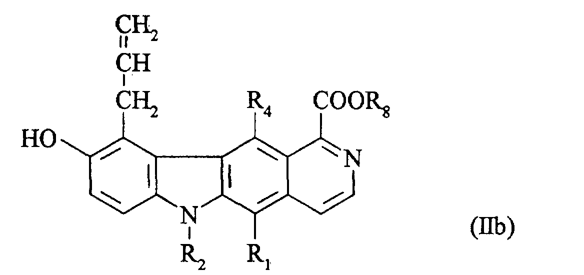 in which R₁, R₂, R₄ and R₈ are as defined above,
. which is reacted with an amine of formula IVa as defined above
. to give a compound of formula Ie: 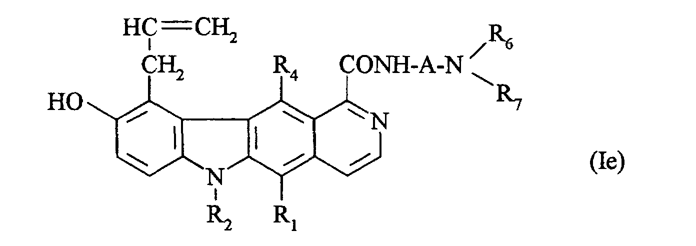 in which R₁, R₂, R₄, R₆, R₇ and A are as defined above;
or
e) a compound of formula IIb defined above is hydrogenated to give a compound of formula IIc: 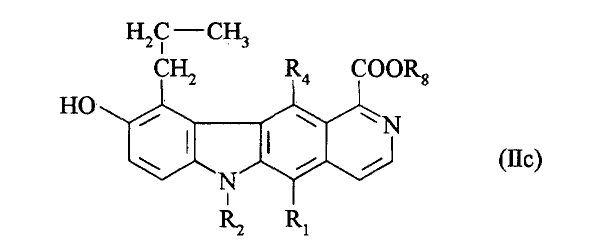 in which R₁, R₂, R₄ and R₈ are as defined above,
. which is reacted with an amine of formula IVa as defined above
. to give a compound of formula If: 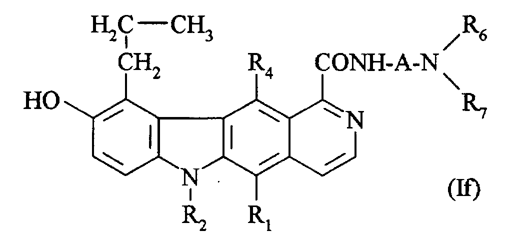 in which R₁, R₂, R₄, R₆, R₇ and A are as defined above.

5. Pharmaceutical compositions comprising as active ingredient at least one compound according to any one of claims 1 and 2, in admixture or in association with one or more pharmaceutical excipients or inert, non-toxic carriers.

6. Pharmaceutical compositions according to claim 5 for use in the treatment of cancers.
